# FASCICULE DE BREVET EUROPEEN

(11) **EP 0 749 734 B1**
(45) Date de publication et mention de la délivrance du brevet: **25.09.2002**
(21) Numéro de dépôt: 96420211.3
(22) Date de dépôt: 21.06.1996
(51) Int. Cl.: A61F 2/38

(54) **Prothèse totale d'articulation du genou**
Kniegelenkvollprothese
Total knee joint prosthesis

(30) Priorité: 21.06.1995 FR 9507678
(43) Date de publication de la demande: 27.12.1996
(73) Titulaire: Afriat, Jacques, 11100 Narbonne (FR); DEDIENNE SANTE, 34130 Mauguio (FR)
(72) Inventeur: Afriat, Jacques, 11100 Narbonne (FR); Audouy, Henri, 31750 Escalquens (FR); Nottebaert, Marc, 81660 Pont de l'Arn (FR)
(74) Mandataire: Maureau, Philippe

(56) Documents cités:
- EP-A- 0 186 471
- EP-A- 0 627 203
- EP-A- 0 636 353
- FR-A- 2 685 632
- GB-A- 2 021 419
- US-A- 5 330 534

## Description

La présente invention concerne une prothèse totale d'articulation du genou, destinée à être implantée avec ablation du ligament croisé postérieur.

Une telle prothèse, dite "postéro-stabilisée", est généralement constituée par un élément ancré dans l'extrémité du fémur, qui présente un bouclier reproduisant les condyles fémoraux et la trochlée de l'articulation naturelle, un élément ancré dans l'extrémité du tibia, qui présente une paroi supérieure sensiblement plane et perpendiculaire à l'axe longitudinal du tibia, et un plateau intermédiaire assurant le glissement de ces deux éléments l'un par rapport à l'autre.

Le plateau intermédiaire comprend, du côté de l'élément fémoral, deux cavités glénoïdes latérales recevant les condyles fémoraux, et, du côté de l'élément tibial, une surface sensiblement plane venant prendre appui sur la paroi supérieure de l'élément tibial.

Le plateau comprend en outre un plot en forme de came, destiné à être engagé dans la trochlée de l'élément fémoral, et l'élément fémoral comprend une barre transversale intercondylienne destinée à venir en butée contre le plot. Cette venue en butée empêche la sub-luxation postérieure du tibia, en remplacement du ligament croisé postérieur supprimé.

Les éléments fémoral et tibial sont généralement métalliques, tandis que le plateau intermédiaire est en matériau favorisant le glissement, tel que du polyéthylène à haute densité.

Lors des mouvements de l'articulation, les éléments fémoral et tibial exercent des efforts importants et répétés sur le plateau intermédiaire, appliqués plus ou moins en porte-à-faux. Ces efforts engendrent, à la longue, une usure et un fluage du polyéthylène du plateau, qui sont sources de déséquilibre de l'articulation et de sollicitations anormales des ligaments, et qui peuvent entraîner le descellement des éléments osseux. Ce problème se pose d'autant plus que cette usure et ce fluage augmentent au fur et à mesure que les éléments fémoral et tibial prennent du jeu par rapport au plateau.

De plus, le contact entre le plot du plateau et la barre inter-condylienne de l'élément fémoral survient en milieu de flexion, plus ou moins brutalement, et se produit sur une surface limitée, ce qui contribue à générer une usure du plateau et un risque de descellement des éléments.

Ces prothèses connaissent, en outre, des problèmes d'alignement fémoro-patellaires, de limitation de la mobilité en flexion et d'existance de contraintes en rotation exposant les éléments prothétiques au descellement.

Il existe des prothèses dans lesquelles le plateau est monté pivotant par rapport à l'élément tibial.

Cette mobilité du plateau permet de limiter l'usure et le fluage précités, ainsi que les problèmes d'alignement fémoro-patellaires, de limitation de la mobilité en flexion et d'existance de contraintes en rotation, sans toutefois les éliminer.

Dans le document EP-A-0627203 un jeu est aménagé entre l'élément fémoral et le plateau intermédiaire entre la positon d'extension de la prothèse ef la position intermédiaire de flexion. Un unique pivotement du plateau intermédiaire par rapport à l'élément tibial est prévu.

La présente invention vise à remédier à cet inconvénient essentiel des prothèses de genou, en fournissant une prothèse qui induit une usure minimale du plateau intermédiaire à long terme tout en conservant une mobilité des éléments fémoral et tibial qui corresponde à celle de l'articulation naturelle.

La prothèse qu'elle concerne comprend, de manière connue en soi, un élément ancré dans l'extrémité du fémur, reproduisant les condyles fémoraux, un élément ancré dans l'extrémité du tibia, qui présente une paroi supérieure sensiblement plane et perpendiculaire à l'axe longitudinal du tibia, et un plateau intermédiaire assurant le glissement de ces deux éléments l'un par rapport à l'autre, ce plateau pouvant pivoter par rapport à l'élément tibial.

Selon l'invention, en combinaison :
- les condyles prothétiques de l'élément fémoral ont une courbure en arc de cercle au niveau de leur partie postérieure, et l'élément fémoral présente, entre ces condyles, une paroi cylindrique convexe ayant un axe confondu avec l'axe du cercle dans lequel sont inscrites les parties postérieures des condyles ;
- les cavités glénoïdes du plateau intermédiaire présentent des parties postérieures en arc de cercle, ayant sensiblement le même rayon, au jeu près, que les parties postérieures des condyles et un axe confondu avec celui du cercle dans lequel sont inscrites ces mêmes parties postérieures, tandis que le plateau intermédiaire comprend une nervure médiane saillante dans laquelle est aménagée une portée concave en arc de cercle, ayant sensiblement le même rayon, au jeu près, que la paroi cylindrique convexe de l'élément fémoral et ayant un axe confondu avec celui de cette paroi, et
- le plateau intermédiaire ou l'élément tibial présente un pion cylindrique formant pivot, tandis que, respectivement, l'élément tibial ou le plateau intermédiaire comprend une cavité de section supérieure à la section transversale de ce pion, cette cavité étant destinée à recevoir ce pion avec possibilité de débattement multidirectionnel.

Lorsque l'élément fémoral est placé au-dessus du plateau intermédiaire, les condyles s'engagent étroitement dans les cavités glénoïdes, et la paroi cylindrique convexe de l'élément fémoral vient étroitement au contact de la portée concave inter-condylienne du plateau intermédiaire.

La paroi convexe et la portée concave constituent un véritable "troisième condyle", permettant non seulement une postéro-stabilisation de l'articulation prothétique, mais également d'assurer le parfait guidage de l'élément fémoral sur le plateau intermédiaire, autour d'un axe transversal précis et selon un mouvement de flexion harmonieux, ainsi que le maintien d'une surface de contact importante quel que soit le degré de flexion de l'articulation.

Le pion et la cavité de l'élément tibial et du plateau intermédiaire permettent un débattement multidirectionnel du plateau par rapport à l'élément tibial, afin que le plateau puisse suivre les mouvements antéro-postérieurs et latéraux du fémur par rapport au tibia et reste en permanence au contact étroit de l'élément fémoral. En effet, le haut niveau de congruence fémoro-tibiale rend impossible toute rotation entre le l'élément fémoral et le plateau intermédiaire. Cette rotation est cependant autorisée sans limite entre le plateau intermédiaire et l'élément tibial.

Ainsi, dans la prothèse selon l'invention, les surfaces de contact de l'élément fémoral et du plateau intermédiaire ne subissent aucun mouvement relatif de translation antéro-postérieure ou latérale et aucun mouvement de pivotement autour d'un axe vertical, mais uniquement un mouvement de pivotement autour d'un axe transversal précis.

Il en résulte que les risques d'usure et de fluage du plateau intermédiaire, ainsi que les risques de descellement des éléments osseux, sont fortement réduits par rapport aux prothèses existantes.

De plus, la surface de contact importante entre l'élément fémoral et le plateau intermédiaire permet une bonne répartition des contraintes, contribuant à limiter l'usure.

Le coefficient de friction du plateau intermédiaire sur l'élément tibial est réduit au maximum par un polissage de la face supérieure de l'élément tibial. Les surfaces en contact sont importantes, ce qui permet également de réduire l'usure du plateau.

Le débattement multidirectionnel du plateau intermédiaire, et donc l'auto-positionnement ou auto-centrage de ce plateau par rapport à l'élément tibial permet de limiter les contraintes en rotation, antéro-postérieures ou médio-latérales que subit le plateau intermédiaire. Elle a également les avantages indiqués ci-dessous.

Sur une prothèse hautement congruente le ligament latéral interne se tend en flexion et empêche la flexion complète. La mobilité en rotation et l'auto-centrage du plateau permettent de libérer les tensions ligamentaires latérales, et donc d'augmenter la possibilité de flexion complète de l'articulation.

La mobilité antéro-postérieure permet de restituer un mouvement de roulement-glissement à la prothèse, à savoir un mouvement de glissement pur au niveau de l'interface élément fémoral-plateau intermédiaire, et un mouvement de roulement du fémur par rapport au tibia, limité à quelques millimètres. Lors de la flexion, le plateau intermédiaire avance jusqu'à venue du pion en butée contre la paroi délimitant la partie postérieure de la cavité. La stabilisation postérieure est ensuite assurée par le troisième condyle.

La translation latérale permet, quant à elle, de compenser une erreur de centrage médio-latéral de l'élément fémoral ou tibial.

Avantageusement, la nervure médiane du plateau intermédiaire a une largeur constante, et l'élément fémoral comprend une cage intercondylienne, contenant la portée concave précitée, venant coiffer la nervure sans jeu latéral.

Les parois latérales de cette cage et de cette nervure constituent des moyens de maintien latéral de l'élément fémoral par rapport au plateau intermédiaire, empêchant tout déplacement latéral pouvant être source d'usure.

De préférence, la cavité recevant le pion présente une forme sensiblement ovale dont la longueur est orientée dans la direction antéro-postérieure. Cette forme de la cavité autorise un débattement antéro-postérieur supérieur au débattement latéral, et permet de reproduire le jeu naturel limité du fémur par rapport au tibia.

Avantageusement, les parties antérieures des condyles de l'élément fémoral et les parties antérieures des cavités glénoïdes du plateau intermédiaire sont congruentes.

Lorsque l'articulation est en extension, ces parties antérieures viennent encore augmenter la surface de contact de l'élément fémoral et du plateau intermédiaire. La charge exercée sur l'articulation est ainsi répartie sur une surface particulièrement importante, ce qui contribue également à limiter l'usure du plateau.

Selon une autre caractéristique avantageuse, la face supérieure de l'élément tibial et la face inférieure du plateau intermédiaire sont inclinées d'environ 5° vers l'arrière, de manière à limiter les risques de soulèvement antérieur du plateau intermédiaire lorsque l'articulation est en flexion maximale.

La prothèse selon l'invention peut également comprendre un implant rotulien en forme de dôme, venant en contact congruent avec la trochlée prothétique aménagée dans la face antérieure de l'élément fémoral, dans les différents secteurs de flexion de l'articulation.

En cas de défaut de positionnement en rotation de l'élément tibial, la rotation du fémur réaligne les éléments fémoral et tibial, et recentre la rotule. La sub-luxation externe de la rotule est donc prévenue. Lors de la flexion, les tensions latérales exercées sur l'appareil extenseur sont équilibrées par l'adaptation de la position du plateau intermédiaire.

Pour sa bonne compréhension, l'invention est à nouveau décrite ci-dessous en référence au dessin schématique annexé représentant, à titre d'exemple non limitatif, une forme de réalisation préférée de la prothèse totale d'articulation de genou qu'elle concerne.

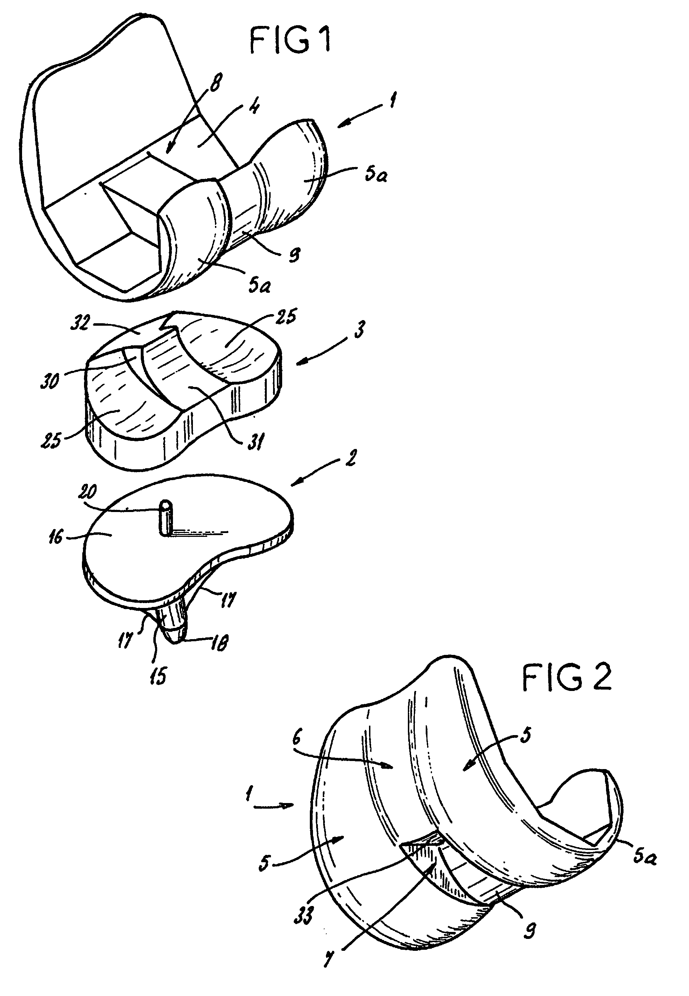

La figure 1 est une vue en perspective éclatée des différents éléments qu'elle comprend ;
la figure 2 est une vue en perspective, sous un autre angle, de son élément fémoral ;
la figure 3 est une vue de la prothèse en coupe selon la ligne III-III de la figure 4, et
la figure 4 en est une vue en coupe selon la ligne IV-IV de la figure 3.

Les figures représentent sous différents angles un élément fémoral 1, un élément tibial 2 et un plateau intermédiaire 3 d'une prothèse d'articulation du genou destinée à être implantée avec ablation du ligament croisé postérieur.

L'élément fémoral 1 est métallique, et a la forme générale d'un bouclier 4.

Sa face externe présente deux parties saillantes 5 reproduisant les condyles fémoraux, une partie intermédiaire 6 en creux, reproduisant la partie antérieure de la trochlée de l'articulation naturelle, une cavité médiane 7 délimitée par une cage intercondylienne 8, et une paroi cylindrique convexe 9, occupant la partie postérieure de la cage 8.

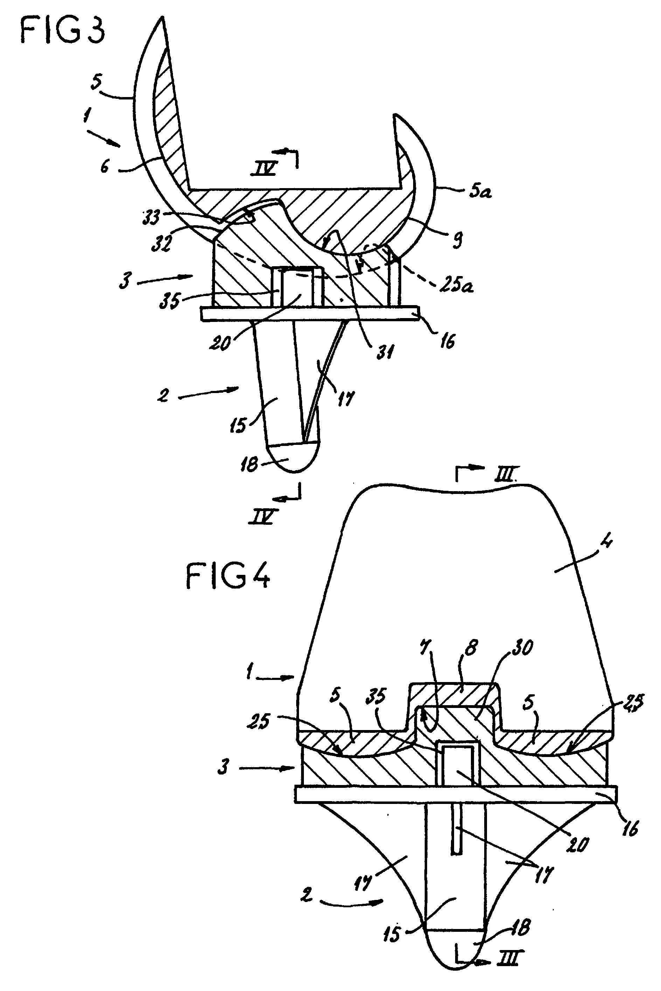

La figure 3 montre plus particulièrement que les condyles prothétiques 5 ont une courbure en arc de cercle au niveau de leur partie postérieure 5a, et que la paroi cylindrique 9 a un axe confondu avec l'axe du cercle dans lequel sont inscrites ces parties postérieures 5a.

Sur sa face interne, destinée à venir au contact de l'os, l'élément fémoral 1 comprend des moyens (non représentés) d'ancrage à l'os. Il peut s'agir entre autres d'une tige médullaire, de pions ou picots d'ancrage ou d'un revêtement poreux favorisant l'ostéo-intégration, tel qu'un revêtement d'hydroxyapatite de calcium.

L'élément tibial 2 est également en matériau métallique.

Il comprend une quille d'ancrage 15, destinée à être engagée dans le canal médullaire de l'os, et une paroi supérieure 16, sensiblement plane, destinée à recevoir le plateau 3.

La quille 15 est solidaire de trois ailettes 17, formant des goussets de renforcement de sa liaison avec la paroi 16 et constituant des parois de stabilisation en rotation de l'élément 2. A sa partie inférieure, cette quille 15 présente un alésage taraudé permettant la mise en place par vissage d'un embout 18, plusieurs embouts de différentes longueurs pouvant être utilisés selon la profondeur de l'ancrage à réaliser.

La paroi 16 présente un pion cylindrique médian 20 faisant saillie perpendiculairement à sa face supérieure. Comme le montre la figure 3, cette paroi 16, avec ce pion 20, sont inclinés d'environ 5° vers l'arrière par rapport à l'axe de la quille 15.

Le plateau intermédiaire 3 est en matériau favorisant le glissement, tel que du polyéthylène à haute densité.

Il comprend, du côté de l'élément fémoral 1, deux cavités glénoïdes latérales 25 recevant les condyles 5, et, du côté de l'élément tibial 2, une surface sensiblement plane, qui est inclinée de 5° vers l'arrière de manière à venir prendre appui sur la paroi 16.

Comme le montre la figure 3 en traits interrompus, les cavités glénoides 25 présentent des parties postérieures 25a en arc de cercle, ayant sensiblement le même rayon, au jeu près, que les parties postérieures 5a des condyles 5, et ayant un axe confondu avec celui de ces mêmes extrémités 5a.

En outre, le plateau intermédiaire 3 comprend une nervure médiane saillante 30, dans la face postérieure de laquelle est aménagée une portée concave 31 en arc de cercle. Cette portée a le même rayon, au jeu près, que la paroi cylindrique 9 et a un axe confondu avec celui de cette paroi 9.

En avant de la paroi 9, la nervure médiane 30 présente une face antérieure 32 légèrement arrondie, et la paroi 33 de l'élément fémoral délimitant le fond de la cavité 7 présente une forme correspondante, cette paroi 33 étant prévue pour venir en appui contre cette face 32 lorsque l'articulation est en extension.

De plus, ainsi que cela apparaît sur la figure 4, la nervure médiane 30 a une largeur constante correspondant, au jeu près, à la largeur de la cavité 7, de sorte que la cage intercondylienne 8 vient coiffer la nervure 30 sans jeu latéral.

Dans sa face inférieure, le plateau intermédiaire comprend une cavité 35 de forme sensiblement ovale, dont la longueur est orientée dans la direction antéro-postérieure de la prothèse. Cette cavité 35 a une section supérieure à la section transversale du pion 20 et peut recevoir ce dernier avec possibilité de débattement multidirectionnel.

Les figures 3 et 4 montrent que les condyles 5 s'engagent étroitement dans les cavités glénoïdes 25, et que la paroi cylindrique convexe 9 vient étroitement au contact de la portée concave 31, lorsque l'élément fémoral 1 est placé au-dessus du plateau intermédiaire 3.

Ces parties 5 et paroi cylindrique 9 peuvent pivoter par rapport à ces cavités glénoides 25 et portée concave 31. La paroi 9 et la portée 31 constituent un véritable "troisième condyle", permettant une postéro-stabilisation de l'articulation, un parfait guidage de l'élément fémoral 1 sur le plateau intermédiaire 3, autour d'un axe transversal précis et selon un mouvement de flexion harmonieux, ainsi que le maintien d'une surface de contact importante quel que soit le degré de flexion de l'articulation.

En outre, la figure 3 montre que les parties antérieures des condyles 5 et des cavités glénoïdes 25 sont congruentes. Lorsque l'articulation est en extension, ces parties antérieures, ainsi que la face antérieure 32 et la face 33, viennent encore augmenter la surface de contact de l'élément fémoral 1 et du plateau intermédiaire 3. La charge exercée sur l'articulation est ainsi répartie sur une surface particulièrement importante.

Les parois latérales de la cage 8 et de la nervure 30 constituent des moyens de maintien latéral de l'élément fémoral 1 par rapport au plateau intermédiaire 3.

Le pion 20 et la cavité 30 permettent un débattement multidirectionnel du plateau 3 par rapport à l'élément tibial 2, afin que le plateau 3 puisse suivre les mouvements antéro-postérieurs et latéraux du fémur par rapport au tibia, et rester en permanence au contact étroit de l'élément fémoral 1.

L'inclinaison vers l'arrière de la paroi supérieure 16 et de la face inférieure du plateau 3 permet, en outre, de limiter les risques de soulèvement antérieur du plateau lorsque l'articulation est en flexion maximale.

Ainsi, dans la prothèse selon l'invention, les surfaces de contact de l'élément fémoral 1 et du plateau intermédiaire 3 ne subissent aucun mouvement relatif de translation antéro-postérieure ou latérale et aucun mouvement de pivotement autour d'un axe vertical, mais uniquement un mouvement de pivotement autour d'un axe transversal précis.

Il en résulte que les risques d'usure et de fluage du plateau intermédiaire 3, ainsi que les risques de descellement des éléments osseux 1,2, sont fortement réduits par rapport aux prothèses existantes.

De plus, la surface de contact importante entre l'élément fémoral 1 et le plateau intermédiaire 3 permet une bonne répartition des contraintes, contribuant à limiter l'usure.

Il va de soi que l'invention n'est pas limitée à la forme de réalisation décrite ci-dessus à titre d'exemple mais qu'elle en embrasse, au contraire, toutes les variantes de réalisation. Ainsi, la prothèse peut notamment comprendre un implant rotulien en forme de dôme, venant en contact congruent avec la trochlée prothétique 6 dans les différents secteurs de flexion de l'articulation.

## Revendications

1. Prothèse totale d'articulation du genou, destinée à être implantée avec ablation du ligament croisé postérieur, du type comprenant un élément ancré dans l'extrémité du fémur, reproduisant les condyles fémoraux, un élément ancré dans l'extrémité du tibia, qui présente une paroi supérieure sensiblement plane et perpendiculaire à l'axe longitudinal du tibia, et un plateau intermédiaire assurant le glissement de ces deux éléments l'un par rapport à l'autre, ce plateau pouvant pivoter par rapport à l'élément tibial, prothèse **caractérisée en ce que** , en combinaison :
- les condyles prothétiques (5) de l'élément fémoral (1) ont une courbure en arc de cercle au niveau de leur partie postérieure (5a), et l'élément fémoral (1) présente, entre ces condyles (5), une paroi cylindrique convexe (9) ayant un axe confondu avec l'axe du cercle dans lequel sont inscrites les parties postérieures (5a) des condyles (5) ;
- les cavités glénoïdes (25) du plateau intermédiaire (3) présentent des parties postérieures (25a) en arc de cercle, ayant sensiblement le même rayon, au jeu près, que les parties postérieures (5a) des condyles (5) et un axe confondu avec celui du cercle dans lequel sont inscrites ces mêmes parties postérieures (5a), tandis que le plateau intermédiaire (3) comprend une nervure médiane saillante (30) dans laquelle est aménagée une portée concave (31) en arc de cercle, ayant sensiblement le même rayon, au jeu près, que la paroi cylindrique convexe (9) de l'élément fémoral (1) et ayant un axe confondu avec celui de cette paroi (9), et
- le plateau intermédiaire (3) ou l'élément tibial (2) présente un pion cylindrique (20) formant pivot, tandis que, respectivement, l'élément tibial (2) ou le plateau intermédiaire (3) comprend une cavité (35) de section supérieure à la section transversale de ce pion (20), cette cavité (35) étant destinée à recevoir ce pion (20) avec possibilité de débattement multidirectionnel.

2. Prothèse de genou selon la revendication 1, **caractérisée en ce que** la nervure médiane (30) du plateau intermédiaire (3) a une largeur constante, et **en ce que** l'élément fémoral (1) comprend une cage intercondylienne (8), contenant la portée concave (31) précitée, venant coiffer la nervure (30) sans jeu latéral.

3. Prothèse de genou selon la revendication 1 ou la revendication 2, **caractérisée en ce que** la cavité (35) recevant le pion (20) présente une forme sensiblement ovale dont la longueur est orientée dans la direction antéro-postérieure.

4. Prothèse de genou selon l'une des revendications 1 à 3, **caractérisée en ce que** les parties antérieures des condyles (5) et des cavités glénoïdes (25) sont congruentes.

5. Prothèse de genou selon l'une des revendications 1 à 4, **caractérisée en ce que** la nervure médiane (30) présente une face antérieure (32) légèrement arrondie, et l'élément fémoral (1) présente une paroi (33) de forme correspondante, cette paroi (33) étant prévue pour venir en appui contre cette face (32) lors que l'articulation est en extension.

6. Prothèse de genou selon l'une des revendications 1 à 5, **caractérisée en ce que** la face supérieure de l'élément tibial (2) et la face inférieure du plateau intermédiaire (3) sont inclinées d'environ 5° vers l'arrière.

7. Prothèse de genou selon l'une des revendications 1 à 6, **caractérisée en ce qu'**elle comprend un implant rotulien en forme de dôme, venant en contact congruent avec la trochlée prothétique (6) aménagée dans la face antérieure de l'élément fémoral (1), dans les différents secteurs de flexion de l'articulation.

## Patentansprüche

1. Kniegelenk-Total-Prothese zur Implantation bei Ablösung des hinteren Kreuzbandes, von der Art, die ein im Ende des Femurs verankertes Element, das die femoralen Kondylen reproduziert, ein im Ende der Tibia verankertes Element, das eine etwa ebene und zur Längsachse der Tibia etwa senkrechte obere Wand aufweist, und eine dazwischenliegende Scheibe aufweist, die die Gleitbewegung dieser beiden Elemente relativ zueinander gewährleistet, wobei sich die Scheibe relativ zu dem tibialen Element drehen kann, **dadurch gekennzeichnet, daß** in Kombination:
- die prothetischen Kondylen (5) des femoralen Elements eine kreisbogenförmige Krümmung in ihrem hinteren Bereich (5a) aufweisen, und daß das femorale Element (1) zwischen den Kondylen eine konvexe zylindrische Wand (9) aufweist, die eine mit der Achse des Kreises, in den die hinteren Bereiche (5a) der Kondylen (5) einbeschrieben sind, zusammenfallende Achse aufweist;
- die Gelenkhöhlungen (25) der dazwischenliegenden Scheibe (3) kreisbogenförmige hintere Bereiche (25a) aufweisen, die etwa, bis auf Spiel, den gleichen Radius aufweisen wie die hinteren Bereiche (5a) der Kondylen (5), und eine mit der Achse des Kreises, in den dieselben hinteren Bereiche (5a) einbeschrieben sind, zusammenfallende Achse aufweisen, während die dazwischenliegende Scheibe (3) eine vorspringende mittlere Rippe (30) aufweist, an der eine kreisbogenförmige konkave Lagerfläche (31) ausgebildet ist, die etwa, bis auf Spiel, denselben Radius wie die konvexe zylindrische Wand (9) des femoralen Elements (1) und eine mit der Achse dieser Wand (9) zusammenfallende Achse aufweist, und daß
- die dazwischenliegende Scheibe (3) oder das tibiale Element (2) einen zylindrischen, ein Drehgelenk bildenden Zapfen (20) aufweist, während entsprechend das tibiale Element (2) oder die dazwischenliegende Scheibe (3) eine Höhlung (35) mit einem größeren Querschnitt als dem Querschnitt des Zapfens (20) aufweist, wobei die Höhlung (35) dazu bestimmt ist, den Zapfen (20) mit der Möglichkeit einer vielgerichteten Verschiebbarkeit aufzunehmen.

2. Kniegelenk-Prothese nach Anspruch 1, **dadurch gekennzeichnet, daß** die mittlere Rippe (30) der dazwischenliegenden Scheibe (3) eine konstante Breite aufweist, und daß das femorale Element (1) ein interkondyläres Gehäuse (8) aufweist, das die vorstehend genannte konkave Lagerfläche (31) einfaßt und die Rippe (30) ohne seitliches Spiel bedeckt.

3. Kniegelenk-Prothese nach Anspruch 1 oder 2, **dadurch gekennzeichnet, daß** die den Zapfen (20) aufnehmende Höhlung (35) eine etwa ovale Form aufweist, deren Langachse in der anteroposterioren Richtung orientiert ist.

4. Kniegelenk-Prothese nach einem der Ansprüche 1 bis 3, **dadurch gekennzeichnet, daß** die vorderen Bereiche der Kondylen (5) und die Gelenkhöhlungen (25) deckungsgleich sind.

5. Kniegelenk-Prothese nach einem der Ansprüche 1 bis 4, **dadurch gekennzeichnet, daß** die mittlere Rippe (30) eine etwas abgerundete Vorderseite (32) aufweist, und daß das femorale Element (1) eine Wand (33) von entsprechender Form aufweist, wobei diese Wand (33) dazu vorgesehen ist, gegen die Seite (32) in Anlage zu kommen, wenn das Gelenk gestreckt ist.

6. Kniegelenk-Prothese nach einem der Ansprüche 1 bis 5, **dadurch gekennzeichnet, daß** die Oberseite des tibialen Elements (2) und die Unterseite der dazwischenliegenden Scheibe (3) um etwa 5° nach hinten geneigt sind.

7. Kniegelenk-Prothese nach einem der Ansprüche 1 bis 6, **dadurch gekennzeichnet, daß** sie ein Kniescheibenimplantat in Gewölbeform aufweist, das mit der prothetischen Trochlea (6), die an der Vorderseite des femoralen Elements (1) ausgebildet ist, in den verschiedenen Beugungsphasen des Gelenks deckungsgleich in Berührung kommt.

## Claims

1. Complete knee joint prosthesis intended to be implanted with ablation of the posterior cruciate ligament, of the type comprising an element which is anchored in the end of the femur and reproduces the femoral condyles, an element which is anchored in the end of the tibia and has an essentially plane upper wall perpendicular to the longitudinal axis of the tibia, and an intermediate plate which provides for the sliding of these two elements in relation to one another, this plate being capable of pivoting in relation to the tibial element, which prosthesis is **characterized in that**, in combination:
- the prosthetic condyles (5) of the femoral element (1) have a curvature in the shape of a circular arc in their rear part (5a), and the femoral element (1) has, between these condyles (5), a convex cylindrical wall (9) which has an axis which coincides with the axis of the circle in which the rear parts (5a) of the condyles (5) lie;
- the glenoid cavities (25) of the intermediate plate (3) have rear parts (25a) in the shape of a circular arc which have essentially the same radius, play excepted, as the rear parts (5a) of the condyles (5) and an axis which coincides with that of the circle in which these same rear parts (5a) lie, while the intermediate plate (3) comprises a central projecting rib (30) in which a concave bearing surface (31) in the shape of a circular arc is arranged, which has essentially the same radius, play excepted, as the convex cylindrical wall (9) of the femoral element (1) and has an axis which coincides with that of this wall (9), and
- the intermediate plate (3) or the tibial element (2) has a cylindrical pin (20) forming a pivot, while the tibial element (2) or, respectively, the intermediate plate (3) comprises a cavity (35) of a section which is greater than the cross section of this pin (20), this cavity (35) being intended to receive this pin (20) with the possibility of multidirectional movement.

2. Knee prosthesis according to Claim 1, **characterized in that** the central rib (30) of the intermediate plate (3) has a constant width, and **in that** the femoral element (1) comprises an intercondylar cage (8) which contains said concave bearing surface (31) and comes to cover the rib (30) without lateral play.

3. Knee prosthesis according to Claim 1 or 2, **characterized in that** the cavity (35) receiving the pin (20) has an essentially oval shape, the length of which is oriented in the anteroposterior direction.

4. Knee prosthesis according to one of Claims 1 to 3, **characterized in that** the front parts of the condyles (5) and of the glenoid cavities (25) are congruent.

5. Knee prosthesis according to one of Claims 1 to. 4, **characterized in that** the central rib (30) has a slightly rounded front face (32), and the femoral element (1) has a wall (33) of corresponding shape, this wall (33) being intended to come to rest against this face (32) when the joint is extended.

6. Knee prosthesis according to one of Claims 1 to 5, **characterized in that** the upper face of the tibial element (2) and the lower face of the intermediate plate (3) are inclined by roughly 5° towards the rear.

7. Knee prosthesis according to one of Claims 1 to 6, **characterized in that** it comprises a patellar implant in the shape of a dome which comes into congruent contact with the prosthetic trochlea (6) arranged in the front face of the femoral element (1), in the various flexion sectors of the joint.
